(19) Europäisches Patentamt
European Patent Office
Office européen des brevets

(11) EP 0 753 507 A1

(12) EUROPÄISCHE PATENTANMELDUNG

(43) Veröffentlichungstag:
15.01.1997 Patentblatt 1997/03

(51) Int. Cl.[6]: C07C 231/02, C07C 235/16

(21) Anmeldenummer: 96110780.2

(22) Anmeldetag: 04.07.1996

(84) Benannte Vertragsstaaten:
BE CH DE ES FR GB IE IT LI

(30) Priorität: 10.07.1995 DE 19525098

(71) Anmelder: HOECHST AKTIENGESELLSCHAFT
65929 Frankfurt am Main (DE)

(72) Erfinder:
- Dierdorf, Andreas, Dr.
60529 Frankfurt (DE)
- Papenfuhs, Theodor, Dr.
60433 Frankfurt (DE)

(54) **Verfahren zur Herstellung von Hydroxycarbonsäureaniliden**

(57) Die vorliegende Erfindung betrifft ein Verfahren zur Herstellung von Hydroxycarbonsäureamiden der allgemeinen Formel (1)

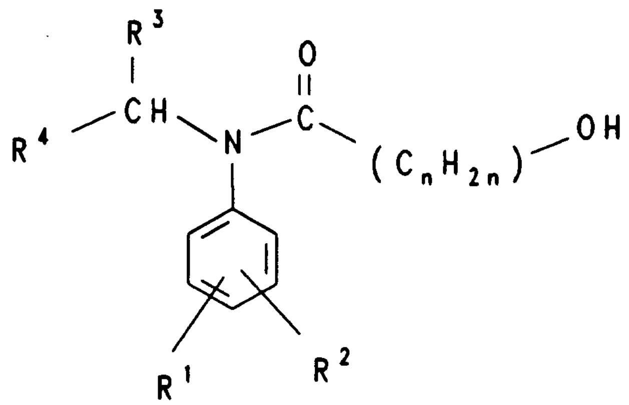

(1)

worin $R^1$ und $R^2$ gleich oder verschieden sind und für Wasserstoff, Halogen, Cyano-, eine geradkettige oder verzweigte Alkyl-, Alkenyl-, Alkinyl-, Alkoxy-Gruppe mit 1 bis 12 Kohlenstoffatomen, eine Aralkyl-Gruppe mit 7 bis 12 Kohlenstoffatomen, eine Cycloalkyl-Gruppe mit 6 bis 12 Kohlenstoffatomen oder eine Aryl-Gruppe mit 6 bis 12 Kohlenstoffatomen stehen, $R^3$ Wasserstoff, eine geradkettige oder verzweigte Alkyl-Gruppe mit 1 bis 4 Kohlenstoffatomen, $R^4$ Wasserstoff, eine geradkettige oder verzweigte Alkyl-Gruppe mit 1 bis 6 Kohlenstoffatomen darstellt oder $R^3$ und $R^4$ gemeinsam mit dem Kohlenstoffatom, an dem sie sitzen, einen fünf- oder sechsgliedrigen Cycloalkanring bilden, und n für eine ganze Zahl von 1 bis 12 steht, indem man ein Anilin der allgemeinen Formel (2)

EP 0 753 507 A1

Printed by Rank Xerox (UK) Business Services
2.13.13/3.4

(Forts. nächste Seite)

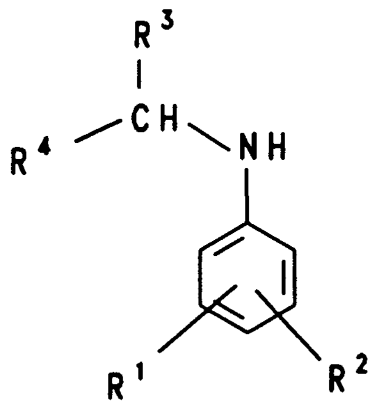

(2)

worin $R^1$, $R^2$, $R^3$ und $R^4$ die in Formel (1) genannte Bedeutung besitzen, mit einer Hydroxycarbonsäure der allgemeinen Formel (3)

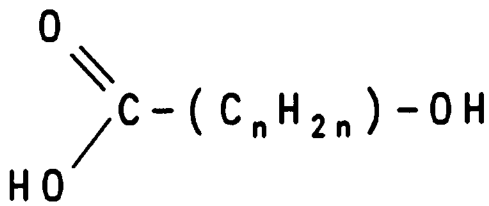

(3)

worin n die in Formel (1) genannte Bedeutung besitzt, in Anwesenheit oder Abwesenheit eines inerten Lösungsmittels bei 150 bis 250°C umsetzt und aus dem Reaktionsgemisch die unter den Reaktionsbedingungen flüchtigen Bestandteile abdestilliert.

## Beschreibung

Die vorliegende Erfindung betrifft ein neues, gegenüber dem Stand der Technik verbessertes Verfahren zur Herstellung von Hydroxycarbonsäureaniliden, ausgehend von den entsprechenden N-Alkylanilinen.

Hydroxycarbonsäureanilide, insbesondere Glykolsäureanilide, stellen eine bedeutsame Stoffgruppe dar und dienen als wichtige Vorprodukte zur Herstellung von Herbiziden (EP-A-300 344) und pharmazeutischen Wirkstoffen (EP-A-284 338, EP-A-363 284) ebenso wie für die Herstellung von Fungiziden (US-4,440,780).

Aufgrund der Bedeutung dieser Stoffgruppe hat es in der Vergangenheit nicht an Versuchen gefehlt, Hydroxycarbonsäureamide und insbesondere Hydroxycarbonsäureanilide auf unterschiedlichen Wegen zugänglich zu machen.

So geht aus der DE-OS-3 038 598 ein Verfahren zur Herstellung von α-Hydroxy-carbonsäureamiden hervor, indem man α-Oxycarbonsäureamide, insbesondere die entsprechenden Formyloxyverbindungen mit Alkoholen in Gegenwart katalytischer Mengen von Alkali- oder Erdalkali-Hydroxiden, Hydrogencarbonaten oder Carbonaten umsetzt. Infolge der dabei ablaufenden Umesterung bilden sich die entsprechenden α-Hydroxycarbonsäureamide. Da die für die Umsetzung benötigten α-Oxy-carbonsäureamide in einem separaten Schritt durch Reaktion von α-Chlorcarbonsäureamiden mit Alkalimetallformiaten hergestellt werden müssen, handelt es sich bei der Herstellung der α-Hydroxycarbonsäureamide - ausgehend von den entsprechenden α-Chlorcarbonsäureamiden - in Wirklichkeit um ein zweistufiges Verfahren, das zudem noch den Nachteil aufweist, daß die Herstellung der α-Oxy-carbonsäureamide in Gegenwart eines quartären Ammoniumsalzes durchgeführt wird. Es ist nämlich bekannt, daß derartige quartäre Ammoniumsalze zu Problemen in der Abwasseraufbereitung führen.

Ein weiteres Verfahren zur Herstellung von α-Hydroxycarbonsäureamiden ist der DE-OS-2 904 490 (US 4,334,073) zu entnehmen. Hierbei setzt man in einer ersten Stufe α-Halogencarbonsäureamide mit einem Alkali- oder Erdalkaliacetat in Gegenwart eines quartären Ammoniumsalzes und gegebenenfalls unter Verwendung eines Verdünnungsmittels zu den entsprechenden α-Acetoxycarbonsäureamiden um und entacyliert die α-Acetoxycarbonsäureamide durch Umsetzung mit einem Alkohol in Gegenwart katalytischer Mengen eines Alkali- oder Erdalkalihydroxides oder eines Alkali- oder Erdalkalicarbonates. Bei diesem Verfahren handelt es sich ebenfalls um einen zweistufigen Prozess, bei dem die Verwendung quartärer Ammoniumsalze ebenfalls zu einer unerwünschten Belastung des Abwassers führt.

Die DE-OS-3 539 394 (EP-A-221 449) betrifft ebenfalls ein zweistufiges Verfahren zur Herstellung von Glykolsäureamiden, indem man Chloracetamide mit Kaliumcarbonat in Gegenwart eines aprotischen Amids als Verdünnungsmittel und gegebenenfalls in Gegenwart eines Phasentransferkatalysators zu symmetrischen Carbonaten umsetzt und diese entweder nach vorheriger Isolierung in einer separaten zweiten Stufe oder ohne intermediäre Isolierung direkt durch Umsetzung mit einem primären Alkohol durch Umesterung in Gegenwart eines Alkalimetallhydroxids entacyliert. In allen Beispielen wird jedoch in Gegenwart eines Phasentransfer-Katalysators gearbeitet. Außerdem lassen die zum Teil recht niedrigen Ausbeuten (22 bis 80 %) noch Wünsche offen.

Die vorstehend geschilderten Verfahren erfordern einen relativ großen Aufwand, da sie die gewünschten Hydroxycarbonsäureamide über zwei separate, nacheinander ablaufende Reaktionsschritte zugänglich machen. Zudem führen die als Phasentransfer-Katalysatoren verwendeten quartären Ammoniumsalze zu Problemen bei den im Verlauf der Umsetzung anfallenden Abfallprodukten. Sie sind insbesondere aufgrund ihrer ungünstigen Eigenschaften im Abwasser unerwünscht. Darüberhinaus werden als Ausgangsmaterial Chlor enthaltende Carbonsäureamide eingesetzt. Die Verwendung chlorhaltiger Stoffe ist jedoch aus Gründen des Umweltsschutzes wie auch hinsichtlich ihrer korrosiven Eigenschaften problematisch.

Jurisic und Zdravskovski beschreiben in Synthetic Communications, 23 (19), 2761 bis 2770 (1993) die Herstellung von Carbonsäureamiden durch Erhitzen einer Mischung eines primären Amins und einer Carbonsäure entsprechend nachfolgender Reaktionsgleichung:

$$R_1NH_2+R_2COOH \xrightarrow{\Delta} R_2NHCOR_2+H_2O$$

Als optimale Reaktionsbedingungen werden Temperaturen von 160 bis 180°C und recht kurze Reaktionszeiten von lediglich 10 bis 30 Minuten angegeben. Es wird ausdrücklich darauf hingewiesen, daß verlängerte Reaktionszeiten die Bildung beträchtlicher Mengen von Teer verursachen können.

Das Verfahren bezieht sich lediglich auf die Umsetzung primärer Amine mit Carbonsäuren. Das erhaltene Reaktionsgemisch wird in heißem Zustand mit Chloroform versetzt und mit Wasser, wäßrigem Kaliumhydroxid und/oder wäßriger Salzsäure extrahiert. Nach Abdampfen des Lösungsmittels wird das Produkt aus Tetrachlorkohlenstoff, Chloroform-Petrolether oder Petrolether umkristallisiert (siehe Seite 2763).

Nachteile dieses Verfahrens bestehen darin, daß das Verfahren zum einen auf den Einsatz primärer Amine beschränkt ist und zum anderen wegen der zu erwartenden Bildung von unerwünschten Abfallprodukten in Form von Dicköl und Teer nur relativ kurze Reaktionszeiten zulässig sind, wobei sichergestellt sein muß, daß innerhalb dieser kurzen Reaktionszeiten überhaupt eine Reaktion in ausreichendem Umfang stattfindet. Von Nachteil ist ferner die arbeitsintensive Aufarbeitung des Reaktionsgemisches, wobei obendrein mit einem chlorhaltigen Lösungsmittel gearbeitet werden muß.

Im Hinblick auf die Bedeutung von Hydroxycarbonsäureaniliden stellt es eine lohnende Aufgabe dar, ein Verfahren zur Herstellung von Hydroxycarbonsäureaniliden bereitzustellen, das die Nachteile der vorstehend genannten Verfahren vermeidet, sich auf einfache Weise und unter Verwendung leicht zugänglicher Ausgangsstoffe und Hilfsstoffe durchführen läßt und darüberhinaus zu einer Verringerung von Abfallprodukten führt.

Gelöst wird diese Aufgabe durch ein Verfahren zur Herstellung von Hydroxycarbonsäureamiden der allgemeinen Formel (1)

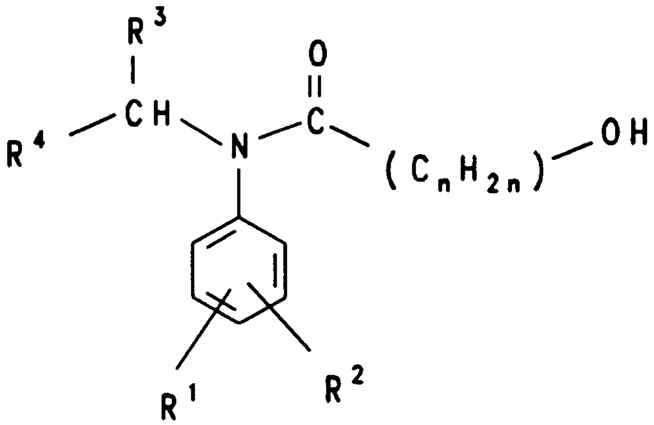

(1)

worin $R^1$ und $R^2$ gleich oder verschieden sind und für Wasserstoff, Halogen, Cyano-, eine geradkettige oder verzweigte Alkyl-, Alkenyl-, Alkinyl-, Alkoxy-Gruppe mit 1 bis 12 Kohlenstoffatomen, eine Aralkyl-Gruppe mit 7 bis 12 Kohlenstoffatomen, eine Cycloalkyl-Gruppe mit 6 bis 12 Kohlenstoffatomen oder eine Aryl-Gruppe mit 6 bis 12 Kohlenstoffatomen stehen, $R^3$ Wasserstoff, eine geradkettige oder verzweigte Alkyl-Gruppe mit 1 bis 4 Kohlenstoffatomen, $R^4$ Wasserstoff, eine geradkettige oder verzweigte Alkyl-Gruppe mit 1 bis 6 Kohlenstoffatomen darstellt oder $R^3$ und $R^4$ gemeinsam mit dem Kohlenstoffatom, an dem sie sitzen, einen fünf- oder sechsgliedrigen Cyclalkanring bilden, und n für eine ganze Zahl von 1 bis 12 steht. Es ist dadurch gekennzeichnet, daß man ein Anilin der allgemeinen Formel (2)

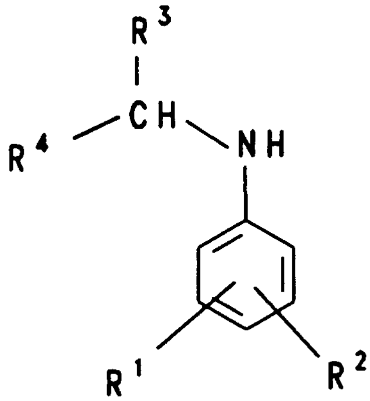

(2)

worin $R^1$, $R^2$, $R^3$ und $R^4$ die in Formel (1) genannte Bedeutung besitzen, mit einer Hydroxycarbonsäure der allgemeinen Formel (3)

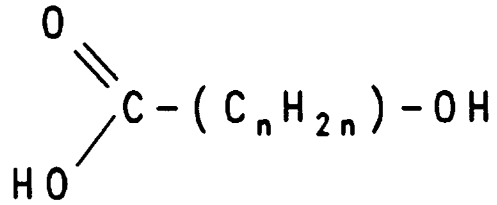

(3)

worin n die in Formel (1) genannte Bedeutung besitzt, in Anwesenheit oder Abwesenheit eines inerten Lösungsmittels bei 150 bis 250°C umsetzt und aus dem Reaktionsgemisch die unter den Reaktionsbedingungen flüchtigen Bestandteile abdestilliert.

Das erfindungsgemäße Verfahren weist mehrere Vorteile auf. Zum einen führt es in einer einzigen Reaktionsstufe zu dem gewünschten Hydroxycarbonsäureanilid und zum anderen kann sowohl auf die Verwendung von Phasentransferkatalysatoren als auch auf Einsatzstoffe, die ein reaktives Chloratom enthalten, nämlich auf α-Chlorcarbonsäureamide oder auf chlorhaltige Lösungsmittel, verzichtet werden. Das erfindungsgemäße Verfahren läßt sich ohne großen technischen Aufwand und unter Verwendung leicht zugänglicher Ausgangsstoffe realisieren.

Im Hinblick auf die in Synthetic Communications 23 (19), 2761 bis 2770 (1993) unter ausschließlicher Verwendung primärer Amine beschriebenen Arbeitsweise ist es überraschend, daß es möglich ist, Aniline der Formel (2), die eine sekundäre Aminogruppe aufweisen, mit einer Hydroxycarbonsäure der Formel (3) umzusetzen. Es war insbesondere nicht zu erwarten, daß sich die im Vergleich zu primären Aminen sterisch generell sperriger gebauten Aniline der Formel (2) überhaupt umsetzen lassen würden und darüberhinaus die gewünschten Reaktionsprodukte trotz vergleichsweise sehr langer Reaktionszeiten in guter Ausbeute und Reinheit zugänglich gemacht werden könnten.

Die Umsetzung verläuft nach folgender Gleichung

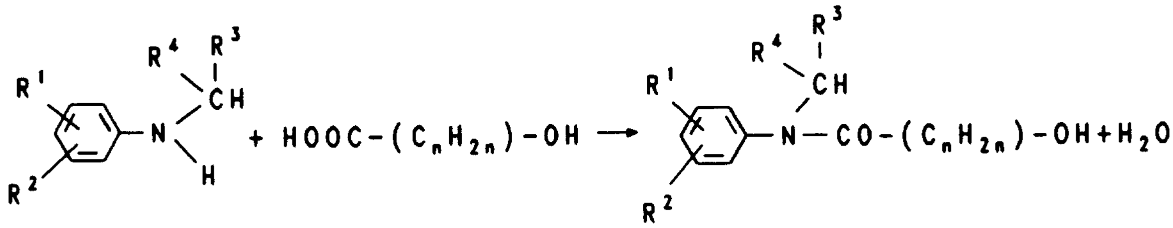

Mit gutem Erfolg läßt sich ein Anilin der Formel (2), worin $R^1$ und $R^2$ gleich oder verschieden sind und für Wasserstoff, Fluor, Chlor, Brom, eine Alkyl- oder Alkoxy-Gruppe mit 1 bis 4 Kohlenstoffatomen stehen, insbesondere ein Anilin der Formel (2), worin $R^1$ und $R^2$ gleich oder verschieden sind und für Wasserstoff, Fluor, Chlor, Brom oder eine Alkyl-Gruppe mit 1 bis 4 Kohlenstoffatomen stehen, bevorzugt ein Anilin der Formel (2), worin $R^1$ und $R^2$ verschieden sind und $R^1$ oder $R^2$ für Wasserstoff steht, einsetzen.

Es empfiehlt sich, ein Anilin der Formel (2), worin $R^3$ für eine Alkyl-Gruppe mit 1 bis 2 Kohlenstoffatomen und $R^4$ für eine geradkettige oder verzweigte Alkyl-Gruppe mit 1 bis 4 Kohlenstoffatomen steht oder $R^3$ und $R^4$ zusammen mit dem Kohlenstoffatom, an dem sie sitzen, eine Cyclopentyl- oder Cyclohexyl-Gruppe bilden, insbesondere ein Anilin der Formel (2), worin $R^3$ für eine Methyl-Gruppe und $R^4$ für eine geradkettige Alkyl-Gruppe mit 1 bis 4 Kohlenstoffatomen steht oder $R^3$ und $R^4$ zusammen mit dem Kohlenstoffatom an dem sie sitzen, eine Cyclohexylgruppe bilden, bevorzugt ein Anilin der Formel (2), worin $R^3$ und $R^4$ für eine Methyl-Gruppe stehen oder zusammen mit dem Kohlenstoffatom, an dem sie sitzen, eine Cyclohexyl-Gruppe bilden, einzusetzen.

Die für das erfindungsgemäße Verfahren benötigten Aniline der Formel (2) lassen sich mit einem vergleichsweise geringen Aufwand herstellen, indem man ein Nitrobenzol der Formel (4)

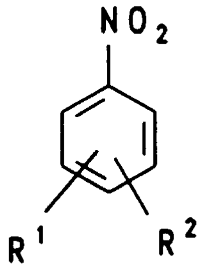

(4)

worin $R^1$ und $R^2$ die im Anilin der Formel (2) genannte Bedeutung besitzen, und eine Carbonylverbindung $R^3$-CO-$R^4$, worin $R^3$ und $R^4$ die im Anilin der Formel (2) genannte Bedeutung besitzen, mit Wasserstoff in Gegenwart eines Hydrierkatalysators umsetzt. Als Hydrierkatalysator eignen sich übliche Nickel, Cobalt, Platin, Palladium enthaltende Katalysatoren, insbesondere Nickel, Cobalt, Platin, Palladium enthaltende Trägerkatalysatoren, wobei als Träger Aluminiumoxid, Bimsstein, Tonerde, $SiO_2$, Kieselgur, Kieselsäure, Aktivkohle oder Mischungen derselben dienen. Es lassen sich jedoch auch trägerfreie Katalysatoren beispielsweise Raney-Nickel, Raney-Cobalt, Platin- oder Palladium in metallischer Form verwenden.

Die vorstehende Umsetzung ist beispielsweise unter Verwendung halogensubstituierter aromatischer Nitroverbindungen in Gegenwart von Metallsulfid-Katalysatoren, die Palladium, Platin, Rhodium, Ruthenium und Kobalt als Metall enthalten, in der US 3,350,450 näher beschrieben. Für eine Reihe derartiger Umsetzungen haben sich auch sulfitierte Edelmetallkatalysatoren, insbesondere sulfitiertes Platin auf Aktivkohle bewährt (EP 0 479 877 B1).

Ein weiterer Vorteil des erfindungsgemäßen Verfahrens besteht darin, daß man ein das Anilin der Formel (2) enthaltendes, durch Umsetzung eines Nitrobenzols der Formel (4)

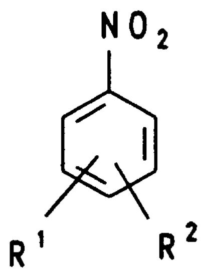

(4)

worin $R^1$ und $R^2$ die in Formel (2) genannte Bedeutung besitzen, und einer Carbonylverbindung $R^3$-CO-$R^4$, worin $R^3$ und $R^4$ die in Formel (2) genannte Bedeutung besitzen, jedoch nicht für Wasserstoff stehen, mit Wasserstoff in Gegenwart eines Hydrierungskatalysators, jedoch in Abwesenheit eines zusätzlichen Lösungsmittels hergestelltes Reaktionsgemisch einsetzt.

Dadurch entfällt eine separate Aufarbeitung des bei dieser Umsetzung anfallenden Reaktionsgemisches, insbesondere ist es nicht erforderlich, das hierbei gebildete Anilin der Formel (2) aus dem Reaktionsgemisch abzutrennen und es in reiner Form mit der Hydroxycarbonsäure der Formel (3) zur Reaktion zu bringen. Man kann das Reaktionsgemisch, gegebenenfalls nach Abtrennung des Hydrierkatalysators und gegebenenfalls nach Abtrennung von Wasser, unmittelbar in das erfindungsgemäße Verfahren einsetzen.

Auf diese Weise ist eine sehr einfache Verfahrensdurchführung, ausgehend von Nitrobenzolen der Formel(4) und Carbonylverbindungen $R^3$-CO-$R^4$, worin $R^3$ und $R^4$ die in Formel (2) genannte Bedeutung besitzen, jedoch nicht für Wasserstoff stehen, sichergestellt,die ohne großen Aufwand für eine separate Aufarbeitung oder Reinigung des Anilins der Formel (2) auskommt.

Dies stellt - wie zuvor erwähnt - einen weiteren Vorteil des erfindungsgemäßen Verfahrens dar.
Ohne Anspruch auf Vollständigkeit zu erheben seien als Beispiele für geeignete Aniline der Formel (2) N-Alkyl-2-methoxyaniline, N-Alkyl-4-methoxyaniline, N-Alkyl-3,5-dimethylaniline, N-Alkyl-2-chloraniline, N-Alkyl-4-chloraniline, N-Alkylaniline und N-Alkyl-4-fluoraniline, worin Alkyl für eine Methyl-, Ethyl-, Propyl-, Isopropyl-, Butyl-, 2-Butyl-, 2-Pentyl- oder eine substituierte oder unsubstituierte Cyclopentyl- oder Cyclohexyl-Gruppe steht, genannt.

Wie eingangs erwähnt, setzt man eine Hydroxycarbonsäure der Formel (3) $HOOC(C_nH_{2n})OH$, insbesondere eine Hydroxycarbonsäure der Formel $HOOC(CH_2)_nOH$, worin also die -$(C_nH_{2n})$-Gruppe für eine -$(CH_2)_n$-Gruppe steht, ein.

In der die Hydroxycarbonsäure betreffenden Formel (3) steht - wie eingangs bereits erwähnt - n für eine ganze Zahl von 1 bis 12, insbesondere für eine ganze Zahl von 1 bis 4, bevorzugt für 1.

Man kann das erfindungsgemäße Verfahren in Anwesenheit oder in Abwesenheit eines inerten Lösungsmittels durchführen. Unter inertem Lösungsmittel wird ein solches verstanden, das unter den Reaktionsbedingungen nicht reagiert, sich also hierbei inert verhält.

Verwendet man ein inertes Lösungsmittel, so sollte dieses einen ausreichend hohen Siedepunkt besitzen.

Ohne Anspruch auf Vollständigkeit seien an dieser Stelle Mesitylen, Decalin, N-Methylpyrrolidon, N,N-Dimethylacetamid und N,N-Diethylacetamid als geeignetes inertes Lösungsmittel genannt. Es lassen sich auch Mischungen inerter Lösungsmittel einsetzen. Das Verfahren ist besonders einfach durchzuführen, wenn man auf ein inertes Lösungsmittel verzichtet. Dies ist in einer Vielzahl von Fällen ohne Nachteil möglich.

Man führt - wie zuvor bereits erwähnt - die Reaktion bei 150 bis 250°C durch. In einer großen Zahl von Fällen hat es sich als ausreichend erwiesen, die Umsetzung bei 160 bis 220, insbesondere 170 bis 190°C durchzuführen.

Üblicherweise setzt man das Anilin der Formel (2) und die Hydroxycarbonsäure der Formel (3) in einem Molverhältnis von 15:1 bis 1:15, insbesondere 10:1 bis 1:10, bevorzugt 5:1 bis 1:5, besonders bevorzugt (4 bis 5):1 ein. Häufig genügt es, die beiden Ausgangsstoffe in stöchiometrischem Verhältnis oder einen der beiden Ausgangsstoffe in einem Überschuß bis zu 300, insbesondere 200 Mol% einzusetzen.

Man entfernt aus dem Reaktionsgemisch die unter den Reaktionsbedingungen flüchtigen Bestandteile mittels Destillation. Dies kann bereits vor Beginn der Reaktion, beispielsweise während des Aufheizens, erfolgen, wobei flüchtige Bestandteile aus den Einsatzstoffen entfernt werden. Es ist auch möglich, diese Destillation später vorzunehmen, wenn die Umsetzung in Gang gekommen ist. Neben flüchtigen Bestandteilen, die auf die Einsatzstoffe zurückgehen, sind jedoch in jedem Falle flüchtige Bestandteile, die während der Umsetzung entstehen, insbesondere das Reaktionswasser, zu entfernen.
Man kann die flüchtigen Bestandteile kontinuierlich oder diskontinuierlich abdestillieren.

Besonders günstig ist es, die unter den Reaktionsbedingungen flüchtigen Bestandteile während der Umsetzung aus dem Reaktionsgemisch abzudestillieren. Als flüchtige Bestandteile destilliert man Wasser und gegebenenfalls Spaltungsprodukte, die während der Umsetzung entstehen, nichtumgesetztes Anilin der Formel (2), nichtumgesetztes Nitrobenzol der Formel (4), nichtumgesetzte Carbonylverbindung sowie deren Reaktionsprodukte ab.

Die flüchtigen Bestandteile kann man bei Unterdruck, Normaldruck oder Überdruck abdestillieren.

Das erfindungsgemäße Verfahren kann man - unabhängig von der Destillation - bei Unterdruck, Normaldruck oder Überdruck durchführen.

Das erfindungsgemäße Verfahren gestaltet sich bei Normaldruck besonders einfach.
Es läßt sich sowohl kontinuierlich als auch diskontinuierlich durchführen. Besonders einfach ist es, das Verfahren diskontinuierlich auszuüben.

Die nachfolgenden Beispiele beschreiben die Erfindung näher, ohne sie hierauf zu beschränken.

Experimenteller Teil

Beispiel 1

a) Herstellung von N-Isopropyl-4-fluoranilin

In einem mit Hubrührer bestückten Autoklaven (Volumen 2 Liter) werden 564,5 g (4 mol) 4-Fluornitrobenzol, 255,6 g (4,4 mol) Aceton und 8 g eines Platin-Katalysators auf Aktivkohle (sulfitiert; 50 % wasserfeucht) vorgelegt und unter Rühren bei 80 bis 90°C und einem Wasserstoffdruck von 6 bis 10 bar umgesetzt. Nach Beendigung der Wasserstoffaufnahme wird abgekühlt, der Autoklav entspannt und der Katalysator mittels Filtration abgetrennt.

Man erhält 812 g eines aus einer wäßrigen Phase und einer organischen Phase bestehenden Reaktionsgemisches. Die wäßrige Phase wird in einem Scheidetrichter abgetrennt.
Die organische Phase (589,2 g) enthält, bestimmt durch Gaschromatographie, 97,4 % N-Isopropyl-4-fluoranilin. Das entspricht einer theoretischen Ausbeute von 94 %.

b) Herstellung von Glykolsäure-N-isopropyl-(4-fluoranilid) ohne Lösungsmittel

In einem mit einem Rührer bestückten Glaskolben (Volumen 250 ml) werden 157 g (1 mol) des aus Beispiel 1a) erhaltenen rohen N-Isopropyl-4-fluoranilins (97,4 %ig) und 19 g (0,25 mol) Glykolsäure 21 Stunden unter Rühren auf 180°C erhitzt. Während der Umsetzung werden die unter den Reaktionsbedingungen flüchtigen Bestandteile, insbesondere das sich bildende Reaktionswasser, abdestilliert.
Nach Abschluß der Umsetzung trennt man überschüssiges N-Isopropyl-4-fluoranilin destillativ ab und reinigt das gebildete Glykolsäure-N-isopropyl-(4-fluoranilid) durch fraktionierte Destillation.
Die Ausbeute beträgt 71,9 %, bezogen auf eingesetzte Glykolsäure, die Reinheit des durch fraktionierte Destillation gewonnen Glykolsäure-N-isopropyl-(4-fluoranilids), liegt bei 98,7 %, bestimmt durch Gaschromatographie.

Beispiel 2

a) Herstellung von N-sek.-Butyl-4-methylanilin

In einem mit Hubrührer bestückten Autoklaven (Volumen 2 Liter) werden 411 g (3 mol) 4-Nitrobenzol, 238 g (3,3 mol) 2-Butanon und 8 g eines Platinkatalysators auf Aktivkohle (sulfitiert; 50 % wasserfeucht) vorgelegt und unter Rühren bei 100°C und einem Wasserstoffdruck von 10 bar umgesetzt. Nach Beendigung der Wasserstoffaufnahme (nach 5,5 Stunden Reaktionszeit) wird abgekühlt, der Autoklav entspannt und der Katalysator mittels Filtration abgetrennt.

Man erhält 698 g eines aus einer wäßrigen Phase und einer organischen Phase bestehenden Reaktionsgemisches. Die wäßrige Phase wird in einem Scheidetrichter abgetrennt.
Die organische Phase enthält, bestimmt durch Gaschromatographie, 21,5 % 4-Methylanilin und 76,7 % N-sek.-Butyl-4-methylanilin.
Man destilliert 4-Methylanilin ab und erhält 266 g Rohprodukt, das zu 98,3 % aus N-sek.-Butyl-4-methylanilin besteht (bestimmt durch Gaschromatographie).

b) Herstellung von Glykolsäure-N-sek.-butyl-(4-methylanilid) ohne Zusatz eines Lösungsmittels

In einem mit einem Rührer bestückten Glaskolben (Volumen 250 ml) werden 163 g (1 mol) des aus Beispiel 2a) erhaltenen rohen N-sek.-Butyl-4-methylanilins und 15,2 g (0,2 mol) Glykolsäure 7 Stunden unter Rühren auf 188°C erhitzt. Während der Umsetzung werden die unter den Reaktionsbedingungen flüchtigen Bestandteile, insbesondere das sich bildende Reaktionswasser, abdestilliert. Nach Abschluß der Umsetzung trennt man überschüssiges N-sek.-Butyl-4-methylanilin destillativ ab und reinigt das gebildete Glykolsäure-N-sek.-butyl-(4-methylanilid) durch fraktionierte Destillation. Das Glykolsäure-N-sek.-butyl-(4-methylanilid) destilliert bei 148 bis 154°C (4 mbar).
Die Ausbeute beträgt 50 %, bezogen auf eingesetzte Glykolsäure, die Reinheit des durch fraktionierte Destillation gewonnenen Glykolsäure-N-sek.-butyl-(4-methylanilids) liegt bei 99,7 %, bestimmt durch Gaschromatographie.
Analytische Daten:
Schmelzpunkt: 46-47°C
$^1$H-NMR (300 MHz, $d_6$-DMSO) $\delta$ 0,89 (t, 3H), 0,98 (d, 2H), 1,23 (sep, 1H), 1,41 (sep, 1H), 2,34 (s, 3H), 3,52 (d, 2H), 4,31 (t, 1H), 4,57 (m, 1H), 7,09 (d, 2H), 7,25(d, 2H); IR (kapillar) 3440, 3050, 2980, 2940, 2880, 1770, 1650, 1620, 1520, 830 cm$^{-1}$; MS (70 eV) m/z 221 ($M^+$), 192, 165, 134 (100 %), 91, 57.

Beispiel 3

a) Herstellung von N-sek.-Butyl-4-ethoxyanilin

In einem mit Hubrührer bestückten Autoklaven (Volumen 2 Liter) werden 501 g (3 mol) 4-Ethoxynitrobenzol, 238 g (3,3 mol) 2-Butanon und 8 g eines Platinkatalysators auf Aktivkohle (sulfitiert; 50 % wasserfeucht) vorgelegt und unter Rühren bei 100°C und einem Wasserstoffdruck von 10 bar umgesetzt. Nach Beendigung der Wasserstoffaufnahme wird abgekühlt, der Autoklav entspannt und der Katalysator mittels Filtration abgetrennt.

Man erhält 729,5 g eines aus einer wäßrigen Phase und einer organischen Phase bestehenden Reaktionsgemisches. Die wäßrige Phase wird in einem Scheidetrichter abgetrennt.
Die organische Phase (577,4 g) enthält, bestimmt durch Gaschromatographie, 0,78 % 4-Ethoxyanilin, 94 % N-sek.-Butyl-4-ethoxyanilin und 3,8 % 4-Ethoxynitrobenzol.
Man destilliert 4-Ethoxyanilin ab und erhält 475 g Rohprodukt, das zu 97,7 % aus N-sek.-Butyl-4-ethoxyanilin besteht (bestimmt durch Gaschromatographie).

b) Herstellung von Glykolsäure-N-sek.-butyl-(4-ethoxyanilid) ohne Zusatz eines Lösungsmittels

In einem mit einem Rührer bestückten Glaskolben (Volumen 500 ml) werden 197,5 g (1 mol) des aus Beispiel 3a) erhaltenen rohen N-sek.-Butyl-4-ethoxyanilin und 15,2 g (0,2 mol) Glykolsäure 8 Stunden unter Rühren auf 180°C erhitzt. Während der Umsetzung werden die unter den Reaktionsbedingungen flüchtigen Bestandteile, insbesondere das sich bildende Reaktionswasser, abdestilliert.

Nach Abschluß der Umsetzung trennt man überschüssiges N-sek.-Butyl-4-ethoxyanilin destillativ ab und reinigt das gebildete Glykolsäure-N-sek.-butyl-(4-ethoxyanilid) durch fraktionierte Destillation. Das Glykolsäure-N-sek.-butyl-(4-ethoxyanilid) destilliert bei 170°C (5,3 mbar).

Die Ausbeute beträgt 33,3 % (16,6 g), bezogen auf eingesetzte Glykolsäure, die Reinheit des durch fraktionierte Destillation gewonnenen Glykolsäure-N-sek.-butyl-(4-ethoxyanilids) liegt bei 99,5 %, bestimmt durch Gaschromatographie.
Analytische Daten:

Schmelzpunkt: 62,5°C
$^1$H-NMR (300 MHz, $d_6$-DMSO) $\delta$ 0,88 (t, 3H), 0,96 (d, 3H)$_1$ 1,31 (m, 2H), 1,33 (t, 3H), 3,51 (d, 2H), 4,04 (q, 2H), 4,36 (t, 1H), 4,57 (m, 1H), 6,97 (d, 2H), 7,12 (d, 2H); IR (KBr) 3410, 3080, 2985, 2940, 1650, 1520, 1250, 590 $cm^{-1}$; MS (70 eV) m/z 251 ($M^+$), 222, 195, 164 (100 %), 136, 108, 41.

Beispiel 4

Herstellung von Glykolsäure-N-methylanilid (ausgehend von reinem N-Methylanilin)

In einem mit einem Rührer bestückten Glaskolben (Volumen 100 ml) werden 42,8 g (0,4 mol) N-Methylanilin und 7,6 g (0,1 mol) Glykolsäure 3,75 Stunden unter Rühren auf 180°C erhitzt. Während der Umsetzung werden die unter den Reaktionsbedingungen flüchtigen Bestandteile, insbesondere das sich bildende Reaktionswasser, abdestilliert.

Nach Abschluß der Umsetzung destilliert man das erhaltene Reaktionsgemisch (46 g) unter reduziertem Druck (2,7 mbar) fraktioniert und erhält folgende Fraktionen:

| | |
|---|---|
| Fraktion 1: | 21,1 g (97,5 % N-Methylanilin; 0,19 % Glykolsäure-N-methylanilid) |
| Fraktion 2: | 5,8 g (98,1 % N-Methylanilin; 0,77 % Glykolsäure-N-methylanilid) |
| Fraktion 3: | 1,6 g (60,0 % N-Methylanilin; 35,9 % Glykolsäure-N-methylanilid) |
| Fraktion 4: | 11,0 g ( 3,2 % N-Methylanilin; 95,4 % Glykolsäure-N-methylanilid) |

Fraktion 4 (Schmelzpunkt: 41,5 bis 46,5°C) entspricht mit 11,0 g einer Ausbeute von 66,7 %, bezogen auf eingesetzte Glykolsäure.

Beispiel 5

Herstellung von Glykolsäure-N-methylanilid (ausgehend von reinem N-Methylanilin und wäßriger Glykolsäure)

In einem mit einem Rührer bestückten Glaskolben (Volumen 250 ml) werden 85,6 g (0,8 mol) N-Methylanilin und 26,7 g einer 57 %igen wäßrigen Glykolsäurelösung (entsprechend 0,2 mol Glykolsäure) 4 Stunden unter Rühren auf 175°C erhitzt. Während der Umsetzung werden die unter den Reaktionsbedingungen flüchtigen Bestandteile, insbesondere das als Lösungsmittel für die Glykolsäure verwendete Wasser und das sich bildende Reaktionswasser, abdestilliert.

Nach Abschluß der Umsetzung destilliert man das erhaltene Reaktionsgemisch unter reduziertem Druck (2,6 mbar) und erhält folgende Fraktionen:

| | |
|---|---|
| Fraktion 1: | 64 g (91,5 % N-Methylanilin; 1,6 % Glykolsäure-N-methylanilid) |
| Fraktion 2: | 23 g (0,2 % N-Methylanilin; 97,2 % Glykolsäure-N-methylanilid) |

Fraktion 2 (Schmelzpunkt: 49 bis 51,5°C) entspricht mit 23,0 g einer Ausbeute von 69,7 %, bezogen auf eingesetzte Glykolsäure.

**Patentansprüche**

1. Verfahren zur Herstellung von Hydroxycarbonsäureamiden der allgemeinen Formel (1)

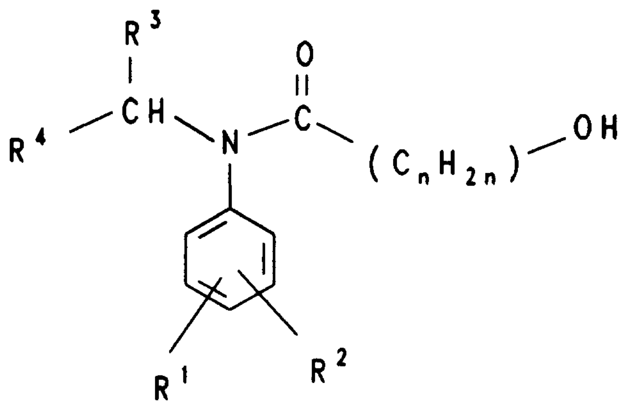

(1)

worin $R^1$ und $R^2$ gleich oder verschieden sind und für Wasserstoff, Halogen, Cyano-, eine geradkettige oder verzweigte Alkyl-, Alkenyl-, Alkinyl-, Alkoxy-Gruppe mit 1 bis 12 Kohlenstoffatomen, eine Aralkyl-Gruppe mit 7 bis 12 Kohlenstoffatomen, eine Cycloalkyl-Gruppe mit 6 bis 12 Kohlenstoffatomen oder eine Aryl-Gruppe mit 6 bis 12 Kohlenstoffatomen stehen, $R^3$ Wasserstoff, eine geradkettige oder verzweigte Alkyl-Gruppe mit 1 bis 4 Kohlenstoffatomen, $R^4$ Wasserstoff, eine geradkettige oder verzweigte Alkyl-Gruppe mit 1 bis 6 Kohlenstoffatomen darstellt oder $R^3$ und $R^4$ gemeinsam mit dem Kohlenstoffatom, an dem sie sitzen, einen fünf- oder sechsgliedrigen Cycloalkanring bilden, und n für eine ganze Zahl von 1 bis 12 steht, dadurch gekennzeichnet, daß man ein Anilin der allgemeinen Formel (2)

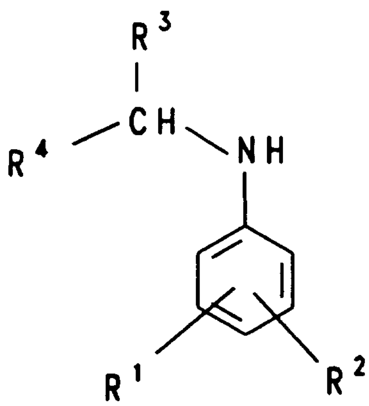

(2)

worin $R^1$, $R^2$, $R^3$ und $R^4$ die in Formel (1) genannte Bedeutung besitzen, mit einer Hydroxycarbonsäure der allgemeinen Formel (3)

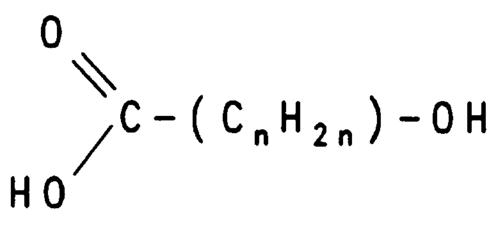

(3)

worin n die in Formel (1) genannte Bedeutung besitzt, in Anwesenheit oder Abwesenheit eines inerten Lösungsmittels bei 150 bis 250°C umsetzt und aus dem Reaktionsgemisch die unter den Reaktionsbedingungen flüchtigen Bestandteile abdestilliert.

2. Verfahren nach Anspruch 1, dadurch gekennzeichnet, daß man ein Anilin der Formel (2), worin $R^1$ und $R^2$ gleich

oder verschieden sind und für Wasserstoff, Fluor, Chlor, Brom, eine Alkyl- oder Alkoxy-Gruppe mit 1 bis 4 Kohlenstoffatomen stehen, einsetzt.

3. Verfahren nach Anspruch 1 oder 2, dadurch gekennzeichnet, daß man ein Anilin der Formel (2), worin $R^1$ und $R^2$ gleich oder verschieden sind und für Wasserstoff, Fluor, Chlor, Brom oder eine Alkyl-Gruppe mit 1 bis 4 Kohlenstoffatomen stehen, einsetzt.

4. Verfahren nach einem oder mehreren der Ansprüche 1 bis 3, dadurch gekennzeichnet, daß man ein Anilin der Formel (2), worin $R^1$ und $R^2$ verschieden sind und $R^1$ oder $R^2$ für Wasserstoff steht, einsetzt.

5. Verfahren nach einem oder mehreren der Ansprüche 1 bis 4, dadurch gekennzeichnet, daß man ein Anilin der Formel (2), worin $R^3$ für eine Alkyl-Gruppe mit 1 bis 2 Kohlenstoffatomen und $R^4$ für eine geradkettige oder verzweigte Alkyl-Gruppe mit 1 bis 4 Kohlenstoffatomen steht oder $R^3$ und $R^4$ zusammen mit dem Kohlenstoffatom, an dem sie sitzen, eine Cyclopentyl-oder Cyclohexyl-Gruppe bilden, einsetzt.

6. Verfahren nach einem oder mehreren der Ansprüche 1 bis 5, dadurch gekennzeichnet, daß man ein Anilin der Formel (2), worin $R^3$ für eine Methyl-Gruppe und $R^4$ für eine geradkettige Alkyl-Gruppe mit 1 bis 4 Kohlenstoffatomen steht oder $R^3$ und $R^4$ zusammen mit dem Kohlenstoffatom an dem sie sitzen,eine Cyclohexylgruppe bilden, einsetzt.

7. Verfahren nach einem oder mehreren der Ansprüche 1 bis 6, dadurch gekennzeichnet, daß man ein Anilin der Formel (2), worin $R^3$ und $R^4$ für eine Methyl-Gruppe stehen oder zusammen mit dem Kohlenstoffatom, an dem sie sitzen, eine Cyclohexyl-Gruppe bilden, einsetzt.

8. Verfahren nach einem oder mehreren der Ansprüche 1 bis 7, dadurch gekennzeichnet, daß man ein das Anilin der Formel (2) enthaltendes, durch Umsetzung eines Nitrobenzols der Formel (4)

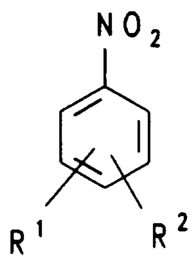

(4)

worin $R^1$ und $R^2$ die in Formel (2) genannte Bedeutung besitzen, und einer Carbonylverbindung $R^3$-CO-$R^4$, worin $R^3$ und $R^4$ die in Formel (2) genannte Bedeutung besitzen, jedoch nicht für Wasserstoff stehen, mit Wasserstoff in Gegenwart eines Hydrierungskatalysators, jedoch in Abwesenheit eines zusätzlichen Lösungsmittels hergestelltes Reaktionsgemisch einsetzt.

9. Verfahren nach einem oder mehreren der Ansprüche 1 bis 8, dadurch gekennzeichnet, daß man eine Hydroxycarbonsäure der Formel (3), worin die $-(C_nH_{2n})$-Gruppe für eine $-(CH_2)_n$-Gruppe steht, einsetzt.

10. Verfahren nach einem oder mehreren der Ansprüche 1 bis 9, dadurch gekennzeichnet, daß man eine Hydroxycarbonsäure der Formel (3), worin n für eine ganze Zahl von 1 bis 4 steht, einsetzt.

11. Verfahren nach einem oder mehreren der Ansprüche 1 bis 10, dadurch gekennzeichnet, daß man eine Hydroxycarbonsäure der Formel (3), worin n für 1 steht, einsetzt.

12. Verfahren nach einem oder mehreren der Ansprüche 1 bis 11, dadurch gekennzeichnet, daß man Mesitylen, Decalin, N-Methylpyrrolidon, N,N-Dimethylacetamid als inertes Lösungsmittel zusetzt.

13. Verfahren nach einem oder mehreren der Ansprüche 1 bis 12, dadurch gekennzeichnet, daß man die Umsetzung bei 160 bis 220°C durchführt.

14. Verfahren nach einem oder mehreren der Ansprüche 1 bis 13, dadurch gekennzeichnet, daß man die Umsetzung

bei 170 bis 190°C durchführt.

15. Verfahren nach einem oder mehreren der Ansprüche 1 bis 14, dadurch gekennzeichnet, daß man das Anilin der Formel (2) und die Hydroxycarbonsäure der Formel (3) in einem Molverhältnis von 15:1 bis 1:15 einsetzt.

16. Verfahren nach einem oder mehreren der Ansprüche 1 bis 15, dadurch gekennzeichnet, daß man das Anilin der Formel (2) und die Hydroxycarbonsäure der Formel (3) in einem Molverhältnis von 10:1 bis 1:10 einsetzt.

17. Verfahren nach einem oder mehreren der Ansprüche 1 bis 16, dadurch gekennzeichnet, daß man das Anilin der Formel (2) und die Hydroxycarbonsäure in einem Molverhältnis von 5:1 bis 1:5 einsetzt.

18. Verfahren nach einem oder mehreren der Ansprüche 1 bis 17, dadurch gekennzeichnet, daß man die unter den Reaktionsbedingungen flüchtigen Bestandteile während der Umsetzung aus dem Reaktionsgemisch abdestilliert.

19. Verfahren nach einem oder mehreren der Ansprüche 1 bis 18, dadurch gekennzeichnet, daß man als flüchtige Bestandteile Wasser und, gegebenenfalls Spaltungsprodukte, die während der Umsetzung entstehen, nichtumgesetztes Anilin der Formel (2), nichtumgesetztes Nitrobenzol der Formel (4), nichtumgesetzte Carbonylverbindung $R^3$-CO-$R^4$ sowie deren Reaktionsprodukte abdestilliert.

20. Verfahren nach einem oder mehreren der Ansprüche 1 bis 19, dadurch gekennzeichnet, daß man die flüchtigen Bestandteile bei Unterdruck, Normaldruck oder Überdruck abdestilliert.

Europäisches Patentamt

# EUROPÄISCHER RECHERCHENBERICHT

Nummer der Anmeldung
EP 96 11 0780

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A,P | EP-A-0 678 502 (HOECHST AG)<br>* das ganze Dokument *<br>--- | 1,8 | C07C231/02<br>C07C235/16 |
| A,P | EP-A-0 695 738 (HOECHST AG)<br>* das ganze Dokument *<br>--- | 1 | |
| A | WO-A-93 22278 (SCHERING AG)<br>* Zusammenfassung *<br>--- | 1 | |
| A | US-A-2 604 481 (RONALD A. HENRY )<br>* das ganze Dokument *<br>--- | 1 | |
| A,D | DE-A-30 38 598 (BAYER AG)<br>* das ganze Dokument *<br>--- | 1 | |
| A<br><br>D | EP-A-0 221 494 (BAYER AG)<br>* Anspruch 1 *<br>& DE-A-35 39 394<br>--- | 1 | |
| A,D | SYNTHETIC COMMUNICATIONS,<br>Bd. 23, Nr. 9, 1993,<br>Seiten 2761-2770, XP000196488<br>BRANKO S. JURSIC ET AL.: "A simple preparation of amides from acids and amines by heating of their mixture"<br>* das ganze Dokument *<br>--- | 1 | RECHERCHIERTE SACHGEBIETE (Int.Cl.6)<br>C07C |
| A | DE-A-32 44 956 (BAYER AG)<br>* Zusammenfassung *<br>----- | 1 | |

Der vorliegende Recherchenbericht wurde für alle Patentansprüche erstellt

| Recherchenort | Abschlußdatum der Recherche | Prüfer |
|---|---|---|
| BERLIN | 22.Oktober 1996 | Rufet, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)